(19) Europäisches Patentamt
European Patent Office
Office européen des brevets

(11) Veröffentlichungsnummer : **0 085 883**
**B1**

(12) # EUROPÄISCHE PATENTSCHRIFT

(45) Veröffentlichungstag der Patentschrift : **15.05.85**

(51) Int. Cl.[4] : **C 07 C 37/04**, C 07 C 39/15

(21) Anmeldenummer : **83100614.3**

(22) Anmeldetag : **25.01.83**

(54) **Verfahren zur Herstellung von Hydroxydiphenylen.**

(30) Priorität : **06.02.82 DE 3204079**

(43) Veröffentlichungstag der Anmeldung : **17.08.83 Patentblatt 83/33**

(45) Bekanntmachung des Hinweises auf die Patenterteilung : **15.05.85 Patentblatt 85/20**

(84) Benannte Vertragsstaaten : **BE CH DE FR GB IT LI**

(56) Entgegenhaltungen :
**FR-A- 1 108 177**
**GB-A- 649 945**
**US-A- 4 243 822**
**HAUBEN-WEYL: "Methoden der organischen Chemie", Band 6, Teil 1C/1, Phenole 1976, Seiten 208-210, Stuttgart, DE.**
**CHEMICAL ABSTRACTS, Band 82, Nr. 5, 3. Februar 1975, Seite 481, Nr. 31122p, Columbus, Ohio, USA**
**CHEMICAL ABSTRACTS, Band 95, Nr. 11, September 1981, Seite 611, Nr. 97365v, Columbus, Ohio, USA**
**CHEMICAL ABSTRACTS, Band 87, Nr. 19, 7. November 1977, Seite 557, Nr. 151840w, Columbus, Ohio, USA**
**CHEMICAL ABSTRACTS, Band 75, Nr. 25, 20. Dezember 1971, Seite 299, Nr. 151519a, Columbus, Ohio, USA**
**CHEMICAL ABSTRACTS, Band 55, Nr. 10, 15. Mai 1961, Spalte 9347, Abschnitt e, Columbus, Ohio, USA**
**CHEMICAL ABSTRACTS, Band 81, Nr. 1, 8. Juli 1974, Seite 287, Nr. 3581y, Columbus, Ohio, USA**

(73) Patentinhaber : **BAYER AG**
**Konzernverwaltung RP Patentabteilung**
**D-5090 Leverkusen 1 Bayerwerk (DE)**

(72) Erfinder : **Mayer, Dietmar, Dr.**
**Hambergerstrasse 15**
**D-5090 Leverkusen 3 (DE)**
Erfinder : **Kramer, Horst-Dieter, Dr.**
**Gabriele-Münterstrasse 6**
**D-5090 Leverkusen 1 (DE)**
Erfinder : **Gabel, Eike, Dr.**
**Eichenhainallee 14**
**D-5060 Bergisch-Gladbach 1 (DE)**
Erfinder : **Köhler, Wilfried, Dr.**
**Jakob-Böhme-Strasse 8**
**D-5000 Köln 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 085 883 B1**

Jouve, 18, rue St-Denis, 75001 Paris, France

**Beschreibung**

Die vorliegende Erfindung betrifft ein Verfahren zur Herstellung von Mono- und Dihydroxy-diphenylen aus den entsprechenden Diphenylsulfonsäuren oder ihren Salzen.

Dihydroxy-diphenyle werden als Ausgangsmaterial für hochwertige Kondensationspolymere, wie Polycarbonate, Polyester und Pulverlacke verwendet, wobei besonders die Eigenschaft der Hochtemperatur-Beständigkeit bemerkenswert ist (DE-OS-3 031 094). Als Kettenabbrecher zur Einstellung der Molekulargewichte sind wegen der strukturellen Ähnlichkeit Monohydroxy-diphenyle einsetzbar. Dihydroxy-diphenyle finden ferner Verwendung als Zwischenprodukt für pharmazeutische Produkte sowie als Stabilisator und Antioxidantien für Kautschuke, Öle und Polymere. 4-Hydroxy-diphenyl (p-Phenylphenol) wird weiterhin als Zwischenprodukt zur Herstellung von Lackharzen, nichtionogenen Emulgatoren und Pflanzenschutzmitteln verwendet (Ullmanns Encyclopädie der technischen Chemie, 4. Auflage, Band 18, Seite 219).

Es ist bekannt, 4-Hydroxy-diphenyl bzw 4,4'-Dihydroxydiphenyl mit Hilfe einer Alkalischmelze aus Diphenyl-4-sulfonsäure bzw. Diphenyl-4,4'-disulfonsäure herzustellen, wobei die Sulfogruppen gegen die Hydroxy-Gruppen ausgetauscht werden. Zu dieser Alkalischmelze werden vielfach die Natriumsalze der genannten Sulfonsäuren zugesetzt, die nach der Sulfonierung von Diphenyl mit überschüssiger Schwefelsäure, Verdünnen des Sulfonierungsansatzes mit Wasser, partieller Neutralisation mit Natronlauge oder Aussalzen mit Kochsalz, anschließender Filtration und Trocknung gewonnen werden.

Beispielsweise sei die JP-Anmeldung 71-30507 genannt, in der 3 bis 5 Mol NaOH pro $SO_3Na$-Gruppe bei einer Temperatur von mindestens 380 °C in druckloser Reaktion umgesetzt werden. Weiterhin sei die DE-OS-3 031 094 genannt, in der ebenfalls in druckloser Schmelzreaktion mit anfangs 2 bis 10 Mol Alkalihydroxid je Sulfonat-Gruppe begonnen wird und dann weiteres Sulfonat in dem Maße zugesetzt wird, daß insgesamt 2 bis 3 Mol Alkalihydroxid, bevorzugt Kaliumhydroxid pro Sulfonat-Gruppe vorliegen. Als Temperatur wird hierbei ein Bereich von 342 bis 350 °C genannt.

Die geschilderten Verfahren setzen teilweise sehr hohe Temperaturen voraus, sind also sehr energieaufwendig und verlangen gegen die hohe Temperaturbeanspruchung spezielle, teure Reaktormaterialien. Die hohen Temperaturen begünstigen auch die Bildung zahlreicher Nebenprodukte, die die Qualität, beispielsweise des 4,4'-Dihydroxydiphenyls, erheblich beeinträchtigen, Da für die erwähnten Polykondensationszwecke meist ein hochgereinigtes Produkt verlangt wird, ist es erforderlich, Anzahl und Konzentration der während der Schmelzreaktion gebildeten Verunreinigungen so gering wie möglich zu halten. Insbesondere beim 4,4'-Dihydroxy-diphenyl ist der Gehalt an monofunktionellen Hydroxylderivaten wegen deren Eigenschaften als Kettenabbrecher in den Polykondensationsreaktionen schädlich.

Um die Reaktionszeiten bei den hohen Temperaturen der geschilderten Verfahren abzukürzen wird hierbei vielfach das teurere Kaliumhydroxid wegen seiner stärkeren Alkalität dem Natriumhydroxid vorgezogen. So beschreibt die bereits erwähnte DE-OS-3 031 094 Ausbeuten bei Verwendung von KOH von etwa 85 % und bei Verwendung von NaOH von nur 75 %. Die Verwendung von KOH wird ebenfalls in GB 2 071 090 beschrieben, worin Diphenyl-di-kaliumsulfonat mit 4,7 Mol KOH pro Äquivalent Kaliumsulfonat-Gruppe bei 335 bis 340 °C während 3 Stunden und dann bei 360 °C für kurze Zeit in einer drucklosen Reaktion erhitzt werden.

Eine weitere Variante zur Herabsetzung der hohen Temperatur der beschriebenen Alkalischmelz-Verfahren besteht in der Verwendung von Zusätzen, die die Schmelztemperaturen und die Viskositäten der Schmelzen herabsetzen sollen. So beschreibt US 2 368 361 ein Verfahren zur Herstellung von Monohydroxy- oder Dihydroxydiphenyl aus den zugehörigen Sulfonsäure-Na-Salzen, bei denen bereits im Anschluß an die Sulfonierung des Diphenyls zur Umsetzung von überschüssiger Schwefelsäure Benzol zur Bildung von Benzolsulfonsäure zugesetzt wird. Diese Benzolsulfonsäure in Form ihres Na-Salzes dient sodann als reaktionsfördernder Zusatz zur Alkalischmelze und wird hierbei in Phenol umgewandelt, das anschließend vom Hydroxy-diphenyl destillativ abgetrennt wird. Dieses Verfahren wird bei 350 bis 370 °C durchgeführt.

In J. Chem. Soc. Japan, Vol. *84*, (1963), S. 143 wird eine Mischschmelze aus etwa 0,7 Mol KOH und 0,5 Mol NaOH zur Durchführung der Alkalischmelze vorgelegt, in die etwa 0,056 Mol Di-kalium-diphenyldisulfonat in kleinen Portionen eingetragen wird. Der Austausch der Sulfonatgruppen gegen OH-Gruppen erfolgt sodann bei 330 °C in einer drucklosen Schmelze. Die Ausbeute beträgt nach zwei Umkristallisationen nur 19 %.

In der JP-Anmeldung 60-8720 wird eine Mischschmelze aus KOH, NaOH und Natriumacetat vorgelegt, in der Diphenylsulfonsäure-Na-Salz drucklos bei 280 bis 300 °C umgesetzt wird.

Eine noch weitere Variante wird in der JP-Anmeldung 71-30508 (zitiert nach C. A., Vol. *75* (1971), 151 518 z) beschrieben, wobei Diphenyl-sulfonsäure-Na-Salz mit NaOH in Gegenwart von o-Phenylphenol oder dessen Salz drucklos bei 360 bis 370 °C umgesetzt wird.

In einer noch weiteren Variante wird in der JP-Anmeldung 71-30509 die Verwendung verschiedener Metallphosphate für die Umwandlung von Diphenyl-sulfonsäuren-Na-Salz in Hydroxy-diphenyle beschrieben. So wird beispielsweise das Diphenyl-Na-sulfonat mit einem 5-fachen molaren Überschuß von wäßriger 45 %iger NaOH in einer 5-stündigen Reaktion bei 380 °C in Gegenwart von 7,8 % Natriumphosphat bzw. Dikalium-hydrogenphosphat mit 98,3 bzw. 98,6 %iger Ausbeute in rohes p-Phenyl-phenol umgewandelt. Läßt man unter sonst gleichen Bedingungen den Phosphatzusatz fort, so sinkt die Ausbeute auf 64,2 %, um erst bei Erhöhung der Reaktionstemperatur auf 400 °C wieder auf 94,5 % anzusteigen.

Die JP-Anmeldung 77-68154 beschreibt ein Verfahren, bei dem Diphenyl-disulfonsäure-Na-salz mit annähernd 5 Mol NaOH pro Na-Sulfonat-Gruppe in Form von 70 %iger NaOH gemischt wird, das Gemisch in einen Rührkessel überführt wird und unter Durchleiten von Stickstoff innerhalb von 6 Stunden auf 330 °C erhitzt wird. Bei dieser Temperatur wird das Reaktionsgemisch sodann für weitere 10 Stunden gehalten. Man erhält hierbei nach Auflösen in Wasser und 30-minütiger Behandlung mit Aktivkohle eine dunkelbraune wäßrige Di-Na-Salzlösung des Dihydroxy-diphenyls, aus der nach Ansäuerung in 80 %iger Ausbeute ein hellbraunes pulverigkristallines Produkt erhalten wird. Dieses für Polymerisationszwecke völlig ungeeignete Dihydroxy-diphenyl muß, entsprechend der Lehre dieser genannten JP-Anmeldung, mit Hydrazin oder Hydrazin-Verbindungen behandelt werden.

Es ist auch bereits versucht worden, die Umsetzung von Diphenyl-Na-Sulfonaten mit wäßriger Natronlauge so durchzuführen, daß dieses Wasser während der Umsetzung im Reaktionsgemisch bleibt. So wird in der JP-Anmeldung 74-16417 die Umsetzung von Diphenyl-Na-sulfonat mit 70 %iger NaOH bei 400 °C in 1 1/2 Stunden zu 93,6 % der theoretischen Ausbeute an rohem p-Phenylphenol beschrieben. Führt man diese Reaktion bei 380 °C durch, so wird in einer 5-stündigen Reaktion nur eine Ausbeute von 82,9 % erzielt. Bei Verwendung von 45 %iger NaOH sinkt die Ausbeute bei 380° Reaktionstemperatur auf 68,1 % und bei 360° Reaktionstemperatur weiter auf 59,2 %. Bei der Umsetzung von Diphenyl-Na-disulfonat zu rohem 4,4′-Dihydroxy-diphenyl wird mit 70 %iger NaOH in 5-stündiger Reaktion bei 380 °C erst eine Ausbeute von 22,3 % erzielt; erst bei einer Temperatur von 440 °C steigt die Ausbeute auf 96,3 %.

Ein noch weiteres Verfahren, wie in der JP-Anmeldung 81-57728 beschrieben, zeigt die Umsetzung von Diphenyl-Na-disulfonat mit 70 %iger Natronlauge in einer 10-stündigen Reaktion bei 330 bis 335 °C unter Durchleiten von Stickstoff. Auch das hierbei erhaltene Rohprodukt muß gereinigt werden, wobei die genannte JP-Anmeldung eine Extraktion mit 1-Heptanol beansprucht. Als Ausbeute werden aus 53,7 Teilen des Disulfonats 19,5 Teile des kristallisierten Dihydroxydiphenyl erhalten, was einer Ausbeute von etwa 70 % der theoretischen Ausbeute entspricht.

Alle beschriebenen Verfahren können somit in Bezug auf die Reinheit und Ausbeute der erhaltenen Produktes oder in Bezug auf die anzuwendenden hohen Temperaturen oder die Umständlichkeit des Verfahrens, beispielsweise bei Anwendung von nachträglich abzutrennenden Zusätzen, nicht befriedigen.

Es wurde nun ein Verfahren zur Herstellung von Hydroxydiphenylen der Formel

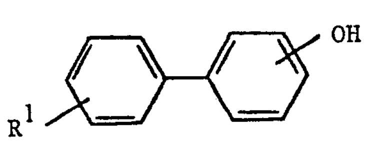

(I)

in der

$R^1$ Wasserstoff oder Hydroxyl bedeutet,

durch Umsetzung von Diphenylsulfonsäuren der Formel

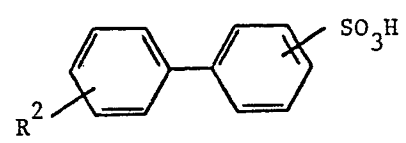

(II)

in der

$R^2$ Wasserstoff oder die Sulfonsäuregruppe bedeutet,

oder ihre Alkalisalze mit Alkalihydroxid bei erhöhter Temperatur und erhöhtem Druck in Gegenwart von Wasser gefunden, das dadurch gekennzeichnet ist, daß man bei einer Temperatur zwischen 280 und 330 °C und einem erhöhten Druck von bis zu 120 bar 3 bis 25 Mol wäßriges Alkalihydroxid mit einer Konzentration von mindestens 50 Gew.-% pro Äquivalent Sulfonatgruppe einsetzt.

Das erfindungsgemäße Verfahren wird bei einer Temperatur zwischen 280 und 330 °C, bevorzugt bei 300 bis 325 °C durchgeführt.

Erfindungsgemäß wird bei einem Druck von bis zu 120 bar, beispielsweise 2 bis 120 bar, bevorzugt bei 5 bis 80 bar, besonders bevorzugt bei 10 bis 40 bar gearbeitet. Diese Drucke können der Eigendruck der Reaktionsmischung sein oder zusätzlich zum Eigendruck durch Aufpressen von Inertgas, beispielsweise Stickstoff, erhöht werden. Der Eigendruck des Reaktionsgemisches ist in einer dem Fachmann im Prinzip geläufigen Weise von den Mischungs- und Konzentrationsverhältnissen im Reaktionsgemisch und der eingestellten Reaktionstemperatur abhängig. Diese Abhängigkeit gilt besonders für den Anteil Wasser im Reaktionsgemisch. Für den Fall, daß ein Druck im unteren Teil der genannten Bereiche eingestellt werden soll, kann dies daher durch Abblasen von Wasserdampf aus dem Reaktionsgemisch über ein Druckhalteventil erfolgen.

Als wäßriges Alkalihydroxid seien beispielsweise wäßrige Lösungen von Natriumhydroxid oder Kaliumhydroxid, bevorzugt Natriumhydroxid, mit einer Konzentration von mindenstens 50 Gew.-% Alkalihydroxid, bezogen auf das Gesamtgewicht der wäßrigen Lösung, genannt. Beispielsweise sei eine Konzentration von 50 bis 96 Gew.-%, bevorzugt von 60 bis 95 Gew.-%, besonders bevorzugt 65 bis 85 Gew.-% genannt. Die Menge des wäßrigen Alkalihydroxids wird hierbei so bemessen, daß 3 bis 25 Mol Alkalihydroxid, bevorzugt 5 bis 12 Mol Alkalihydroxid pro Äquivalent Sulfonatgruppe im Reaktionsgemisch vorhanden sind. Die genannten molaren Mengen Alkalihydroxid beziehen sich hierbei auf Äquivalente an Sulfonatgruppen, wobei hierunter beispielsweise das Natrium- oder das Kaliumsalz einer Diphenylsulfonsäure der Formel (II) genannt sei.

Die Herstellung von Diphenylsulfonsäuren der Formel (II) geschieht in bekannter Weise durch Sulfonieren von Diphenyl mit Oleum oder Schwefelsäure (Liebigs Annalen der Chemie, *207* (1881), 320, besonders S. 336 ; US 2 368 361). Die hierbei erhältlichen Sulfonsäuren der Formel (II) werden sodann zu den entsprechenden Sulfonaten in bekannter Weise neutralisiert. Diese Neutralisation kann außerhalb des Reaktionsgemisches für das erfindungsgemäße Verfahren erfolgen, so daß in das erfindungsgemäße Verfahren ein bereits neutralisiertes Sulfonat eingesetzt wird. Die Neutralisation zum Sulfonat kann jedoch auch im erfindungsgemäßen Verfahren erfolgen, so daß man eine freie Sulfonsäure der Formel (II) in das erfindungsgemäße Verfahren einsetzt. Erfindungsgemäß ist es weiterhin möglich, die Sulfonsäure der Formel (II) oder ihre Alkalisalze in trockner oder wasserfeuchter Form oder in Form ihrer wäßrigen Lösung einzusetzen. Erfindungsgemäß können nun entweder reine Sulfonsäuren der Formel (II) oder Sulfiergemische eingesetzt werden, die solche Sulfonsäuren der Formel (II) neben noch nicht umgesetzter Schwefelsäure und gegebenenfalls neben noch nicht umgesetztem $SO_3$ enthalten. Die zuletztgenannte Variante des erfindungsgemäßen Verfahrens unter Einsatz von noch nicht aufgearbeiteten Sulfiergemischen ist bevorzugt.

Da erfindungsgemäß die molare Menge wäßriges Alkalihydroxid auf die Äquivalente an Sulfonatgruppen bezogen sind, muß beim Einsatz freier Sulfonsäuren bzw. beim Einsatz von solche Sulfonsäuren enthaltenden Sulfiergemischen eine zusätzliche Menge Alkalihydroxid zugefügt werden, die zur vollständigen Neutralisation aller vorhandenen sauren Gruppen ausreicht.

Als Sulfonsäuren der Formel (II) für das erfindungsgemäße Verfahren seien beispielsweise genannt : 4-Diphenylmonosulfonsäure, 2-Diphenylmonosulfonsäure, 3-Diphenylmonosulfonsäure, 4,4'-Diphenyldisulfonsäure, 3,4'-Diphenyldisulfonsäure, 3,3'-Diphenyldisulfonsäure, in bevorzugter Weise 4-Diphenylmonosulfonsäure und 4,4'-Diphenyldisulfonsäure.

Das erfindungsgemäße Verfahren kann beispielsweise wie folgt durchgeführt werden :

In einen Autoklaven legt man eine hochkonzentrierte wäßrige Lösung eines Alkalihydroxids vor und trägt das feuchte oder trockene Alkalisalz einer Diphenylmonosulfonsäure bzw. einer Diphenyldisulfonsäure oder eine wäßrige Lösung eines solchen Alkalisalzes allmählich ein. Der Autoklav wird dann verschlossen und hochgeheizt. Das Reaktionsgemisch wird dabei erfindungsgemäß umgesetzt. Im allgemeinen benötigt man für die Umsetzung 3 bis 25 Stunden. Nach Beendigung der Reaktion wird die Schmelze abgekühlt und in Wasser aufgenommen. Zur Herstellung des freien Hydroxydiphenyls bzw. Dihydroxydiphenyls säuert man das Reaktionsgemisch mit einer Mineralsäure, beispielsweise Salzsäure, an.

In einer besonderen Ausführungsform ist das erfindungsgemäße Verfahren dadurch gekennzeichnet, daß man

a) ein technisches, Schwefelsäure und gegebenenfalls Wasser enthaltendes Diphenylsulfonsäure- bzw. Diphenyldisulfonsäure-Gemisch, das mindestens 80 Gew.-% Diphenylsulfonsäure bzw. Diphenyldisulfonsäure, bezogen auf die insgesamt vorhandenen organischen Bestandteile, und 1 bis 60 Gew.-% Schwefelsäure sowie gegebenenfalls 3 bis 30 Gew.-% Wasser, bezogen auf die Gesamtmasse des Sulfiergemisches, enthält, in die Reaktion einsetzt,

b) dieses Gemisch, gegebenenfalls als wäßrige Lösung, mit so viel mindestens 50 gew.-%igem wäßrigem Alkalihydroxid bei erhöhter Temperatur und gegebenenfalls erhöhtem Druck vermischt, das nach Neutralisation der Schwefelsäure und der gesamten Sulfonsäuregruppen pro Äquivalent Sulfonatgruppe 3 bis 25 Mol Alkalihydroxid vorliegen,

c) diese alkalische Reaktionsmischung bei Temperaturen im Bereich zwischen 280 und 330 °C und bei einem Druck von bis zu 120 bar umsetzt, und

d) die alkalische Reaktionsmischung, gegebenenfalls nach Verdünnen mit Wasser, mit einer Mineralsäure bis zu einem pH-Wert von kleiner als 8 versetzt und das Hydroxydiphenyl der Formel (I) bei einer Temperatur von beispielsweise – 5 °C bis + 100 °C isoliert.

Die erfindungsgemäß einsetzbaren technischen Sulfiergemische können beispielsweise die folgende Zusammensetzung haben :

1. Beispiel eines technischen Diphenyl-4,4'-disulfonsäure-Gemisches :

Mindestens 30 Gew.-%, beispielsweise 30 bis 80 Gew.-% Diphenyl-4,4'-disulfonsäure,

0 bis 8 Gew.-% Diphenyl-3,4'-disulfonsäure,
0 bis 5 Gew.-% Diphenyl-3,3'-disulfonsäure,
0 bis 2 Gew.-% Diphenyl-4-sulfonsäure,
0 bis 54 Gew.-% Schwefelsäure,
20 bis 1 Gew.-% Wasser.

2. Beispiel eines technischen Diphenyl-4-sulfonsäure-Gemisches :

Mindestens 4,5 Gew.-%, beispielsweise 4,5 bis 80 Gew.-% Diphenyl-4-sulfonsäure,
0 bis 5 Gew.-% Diphenyl-2-sulfonsäure,
0 bis 15 Gew.-% Diphenyl-4,4'-disulfonsäure,
0 bis 1 Gew.-% Diphenyl,
15 bis 0,5 Gew.-% Schwefelsäure,
5 bis 74 Gew.-% Wasser.

Diese technischen Sulfiergemische können gegebenenfalls bis zu gleichen Teilen mit Wasser verdünnt und sodann erfindungsgemäß eingesetzt werden. Solche technischen Diphenyl-4-sulfonsäure bzw. Diphenyl-4,4'-disulfonsäure-Gemische können beispielsweise in der oben dargestellten Weise durch Sulfonierung von Diphenyl mit Schwefelsäure, die gegebenenfalls Wasser oder freies $SO_3$ enthält, bei erhöhter Temperatur hergestellt werden.

Nach dem erfindungsgemäßen Verfahren können Sulfiergemische, wie beispielsweise die vorgenannten, direkt und ohne Zwischenisolierung der Diphenyl-4-sulfonsäure bzw. der Diphenyl-4,4'-disulfonsäure oder deren Alkalisalze eingesetzt werden.

Im Falle der Diphenyl-4,4'-disulfonsäure kann es auch zweckmäßig sein, aus dem Sulfiergemisch einen Teil der überschüssigen Schwefelsäure vorher abzutrennen, indem man beispielsweise das Sulfiergemisch mit Wasser verdünnt und die ausfallende Diphenyl-4,4'-disulfonsäure in Form einer nur wenig Schwefelsäure und organische Nebenprodukte enthaltenden Paste beispielsweise durch Filtration isoliert.

Im Falle der Diphenyl-4-sulfonsäure kann es zweckmäßig sein, aus dem Sulfiergemisch nach Verdünnen mit Wasser das nicht umgesetzte Diphenyl ganz oder teilweise beispielsweise mittels Wasserdampf-Destillation abzutrennen.

Beim erfindungsgemäßen Einsatz eines Sulfiergemisches kann dieses ohne Zwischen-Abkühlung mit der Temperatur der vorangegangenen Sulfierreaktion in die erfindungsgemäße Umsetzung mit wäßrigem Alkalihydroxid eingesetzt werden. Hierbei wird die gesamte in der Sulfiermasse enthaltene Wärmemenge in den erfindungsgemäßen verfahrensschritt eingebracht, so daß die Energie, die zum Erwärmen und Aufschmelzen der organischen Komponente anderenfalls aufgebracht werden müßte, eingespart werden kann. Weiterhin kommt bei der direkten Vermischung des technischen Sulfiergemisches mit dem wäßrigen Alkalihydroxid zusätzliche Wärmemenge aus der Neutralisationsreaktion der Schwefelsäure und der Sulfogruppen in das Reaktionsgemisch des erfindungsgemäßen Verfahrens, wodurch sich ein äußerst wirtschaftliches Verfahren zur Herstellung von Hydroxydiphenyl der Formel (I) erschließt.

Dies ist überraschend, weil bei allen bisher bekannt gewordenen Verfahren die Zwischenisolierung der Diphenylmono- bzw. Diphenyl-disulfonsäure in Form ihres Alkalisalzes für notwendig gehalten wurde, weil dieses Salz als Feststoff auf einfache Weise mit dem Alkalihydroxid gemischt werden kann und weil bei der Zwischenisolierung gleichzeitig eine für notwendig gehaltene Reinigung durchgeführt wird. Bei dieser für notwendig gehaltenen Reinigung wird vor allem Schwefelsäure aus dem überschüssigen Sulfierreagenz und ein Teil der bei jeder Sulfierung nach einem wirtschaftlichen Sulfierverfahren unvermeidlichen Nebenprodukte, beispielsweise isomere Diphenylsulfonsäure und andere organische Nebenprodukte, die bei der Reinigung in der Mutterlauge in Lösung bleiben, entfernt werden.

Die bevorzugte Variante des erfindungsgemäßen Verfahrens unter Einsatz von beispielhaft beschriebenen Sulfiergemischen zeigt demnach folgende Vorteile :

a) der zusätzliche Verfahrensschritt der Alkalisalzbildung der Sulfonsäuren ist entbehrlich ;

b) die zur Zwischenisolierung und Trocknung solcher Alkalisalze notwendige Bereitstellung und nachträgliche Verdampfung großer Wassermengen mit dem zugehörigen Energieaufwand sind entbehrlich ;

c) die bei herkömmlichen Reinigungsverfahren zur Zwischenisolierung der genannten Alkalisalze in den Mutterlaugen verbleibenden beträchtlichen Mengen an Diphenylsulfonsäure verbleiben im Verfahren und erhöhen dadurch die Gesamtausbeute ;

d) bei Zwischenisolierungsverfahren nach dem Stande der Technik entstehen Abwasserabläufe, die im Falle der teilweisen Neutralisation und besonders im Falle der Aussalzung solcher Alkalisalze mit Kochsalz beträchtliche Mengen an Schwefelsäure (Dünnsäure) enthalten ; solche Abwasserabläufe werden erfindungsgemäß verhindert ;

e) die obengenannten Wärmeenergien, die im rohen Sulfoniergemisch enthalten sind, bleiben erhalten.

Die Vermischung der beispielhaft genannten technischen Sulfierschmelzen von Diphenylsulfonsäuren mit dem wasserhaltigen Alkalihydroxid kann auf verschiedene Weise erfolgen, um die obengenannten Wärmemengen im Reaktionssystem zu halten und vorteilhaft auszunutzen :

1. Die unverdünnte, technische Diphenylsulfonsäure-Sulfierschmelze wird beispielsweise in auf Siedetemperatur erhitzte, vorgelegte wäßrige Natronlauge der obengenannten Konzentration eindosiert, wobei ohne äußere Wärmezufuhr kontinuierliche Wasser abdestilliert wird und der Ansatz dadurch aufkonzentriert wird. Eine solche Wasserdestillation kann sich aber auch an die Vorvermischung anschließen.

2. Die Diphenylsulfonsäure-Sulfierschmelze wird unter Druck in vorgelegte geschmolzene wäßrige Natronlauge der oben angegebenen Konzentration eingepumpt oder eingedrückt, wobei allein durch Ausnutzung der dabei freiwerdenden Wärmemengen eine für die folgende Umsetzung zu dem Hydroxydiphenyl der Formel (I) günstige Reaktionstemperatur, wie sie oben genannt wurde, erreicht wird. Falls bei dieser Verfahrensvariante eine Begrenzung des gleichzeitig ansteigenden Druckes erzielt werden soll, kann dies durch Abblasen von Wasserdampf über ein Druckhalteventil geschehen.

3. Die unverdünnte technische Sulfierschmelze und das wäßrige Alkalihydroxid werden simultan in ein Mischrohr eingepumpt, wobei das Mischrohr so ausgelegt ist, daß allein durch die bewirkte turbulente Strömung eine vollständige Vermischung erfolgt. Gegebenenfalls kann die Vermischung auch durch Einbauten im Mischrohr unterstützt werden, jedoch ist diese Maßnahme nicht unbedingt erforderlich. Beispielsweise können in einem Mischrohr von 500 mm Länge und 5 mm Durchmesser in einer Stunde 2 000 bis 5 000 ml technische Sulfierschmelze mit den zugehörigen Mengen an wäßriger Natronlauge vermischt und in ein Reaktionsgefäß gefördert werden. Bei geeigneter Wahl der Ausgangstemperaturen der zu mischenden Komponenten, beispielsweise 90 °C bis 130 °C für die Sulfierschmelze und 80 °C bis 110 °C für die Natronlauge, wird am Ende des Mischrohres eine für die Umsetzung zu dem Hydroxydiphenyl der Formel (I) günstige Reaktionstemperatur zwischen 280 und 330 °C erreicht. Hierdurch kann die Umsetzung zum Hydroxydiphenyl der Formel (I) gegebenenfalls mindestens teilweise bereits in diesem Mischrohr durchgeführt werden. Es kann aber auch vorteilhaft sein, die Vermischung bei einer niedrigeren Temperatur vorzunehmen und den resultierenden Wasserdampfdruck durch Abblasen von Wasserdampf in der geschilderten Weise zu begrenzen. Dieses Verfahren stellt somit ein teilkontinuierliches Verfahren dar, bei dem die Vermischung im Mischrohr der kontinuierliche Verfahrensteil und die folgende Vervollständigung der Umsetzung in einem Autoklaven den diskontinuierlichen Teil darstellt.

Die Aufarbeitung und Isolierung des freien Hydroxydiphenyls der Formel (I) erfolgt nach gebräuchlichen Methoden, beispielsweise durch Versetzen mit einer starken Mineralsäure, beispielsweise Salzsäure oder Schwefelsäure, bis zu einem pH-Wert von kleiner als 8, vorzugsweise 6,5 bis 7,5. Danach kann das erhaltene Gemisch mit einer zur Auflösung der anorganischen Salze notwendigen Menge Wasser verdünnt werden und das gewünschte Hydroxydiphenyl in hoher Reinheit ausgefällt werden. Für diese Ausfällung wird beispielsweise eine Temperatur von etwa − 5 °C bis etwa + 100 °C eingestellt.

Im erfindungsgemäßen Verfahren werden die Hydroxydiphenyle der Formel (I) in hoher Ausbeute und in hoher Reinheit erhalten.

## Beispiel 1

Im Inconel-Autoklaven wurden

3 161 g wäßrige 81-%ige Natronlauge (64 Mol) vorgelegt und unter Rühren

1 005 g 100 % (3,2 Mol) Diphenyldisulfonsäure als Natriumsalz eingetragen. Der Autoklav wurde druckdicht verschlossen und 15 Stunden auf 320 °C gehalten. Es stellte sich ein Druck von 8-15 bar ein. Nach Ablauf der Reaktionszeit ließ man auf 280 °C abkühlen und pumpte

ca. 1 000 ml Wasser ein. Nach Abkühlen wurde die Suspension aus dem Autoklaven entnommen. Der Autoklav wurde mit Wasser nachgespült und der Gesamtansatz mit Wasser auf 7 Ltr. aufgefüllt. Zur Isolierung des 4,4'-Dihydroxydiphenyls wurde durch Zugabe von 30-%iger Salzsäure bis zu einem pH-Wert von 7-7,5 neutralisiert. Das ausgefällte Produkt wurde abgesaugt und mit Wasser gewaschen. Die erhaltene Feuchtware wurde im Vakuum bei 100 °C getrocknet. Reinigung erfolgte durch Sublimation.

Analyse (gaschromatographisch) :
98,8-99,0 % 4,4'-Dihydroxydiphenyl
0,9-1,2 % m- und p-Phenylphenol
Ausbeute an 4,4'-Dihydroxydiphenyl sublimiert : 96 % der theoretischen Ausbeute.

## Beispiel 2

Im Inconel-Autoklaven wurden

3 161 g wäßrige 81-%ige Natronlauge (64 Mol) vorgelegt und unter Rühren

749 g 100 % (3,2 Mol) Diphenyl-4-sulfonsäure als Natriumsalz eingetragen. Der Autoklav wurde druckdicht verschlossen und 15 Stunden auf 320 °C gehalten. Es stellte sich ein Druck von 8-15 bar ein. Nach Ablauf der Reaktionszeit ließ man auf 280 °C abkühlen und arbeitete den Reaktionsansatz wie in Beispiel 1 auf.

Analyse (gaschromatographisch) :
99,5-99,6 % 4-Hydroxydiphenyl
0,2 % 4,4'-Dihydroxydiphenyl
Erstarrungspunkt : 164-164,2 °C
Ausbeute an 4-Hydroxydiphenyl sublimiert : 97 % der theoretischen Ausbeute.

Beispiel 3

In einem 1,3-Ltr.-Nickelautoklaven wurden bei 110 °C 693 g wäßrige 75 %ige Natronlauge (= 13 Mol) vorgelegt.

In einem VA-Autoklaven wurden gleichzeitig 479 g Diphenyl-4,4'-disulfonsäure-Sulfiergemisch (1,05 Mol Diphenyl)4,4'-disulfonsäure) folgender Zusammensetzung :

| | | |
|---|---|---|
| | 68,7 | Gew.-% Diphenyl-4,4'-disulfonsäure |
| ca. | 0,5 | Gew.-% Diphenyl-3,4'-disulfonsäure |
| | 0,05 | Gew.-% Diphenyl-4-monosulfonsäure |
| | 5,8 | Gew.-% $H_2SO_4$ |
| | 25,0 | Gew.-% $H_2O$ |

bei 90 °C aufgeschmolzen und mit 20 bar Stickstoff über eine beheizte Leitung innerhalb von ca. 30 Sekunden unter die Oberfläche der intensiv gerührten Natronlauge gedrückt. Die Temperatur im Nickelautoklaven stieg auf 186 °C, der Druck auf 22 bar an. Eine nachfolgende Analyse gab an, daß 0,99 Mol der Sulfierschmelze hierbei zur Reaktion gebracht wurden. Die Reaktionsmischung wurde auf 320 °C erhitzt und 14 h bei dieser Temperatur nachgerührt. Der durch den überlagerten $N_2$-Druck erhöhte Eigendruck stieg bis aus 64 bar an. Nach Abkühlen auf 200 °C wurden in die Reaktionsmischung 400 ml Wasser eingepumpt. Nach weiterem Abkühlen auf 20 °C wurde eine helle, feinkörnige, gut rührbare Suspension erhalten, deren Zusammensetzung mittels Hochdruckflüssigkeitschromatographie analysiert wurde.

In der Suspension waren enthalten :

169,5 g 4,4'-Dihydroxy-diphenyl = 92 % der theoretischen Ausbeute, bezogen auf eingedrückte Diphenyl-4,4'-disulfonsäure und 2,3 g 4-Hydroxy-diphenyl.

Die Aufarbeitung erfolgte wie in Beispiel 1. Man erhielt 169,9 g 98,8-%iges 4,4'-Dihydroxydiphenyl = 91 % der theoretischen Ausbeute. Das Produkt enthielt 1,2 Gew.-% 4-Hydroxydiphenyl.

Beispiel 4

In einem 1,3 Ltr. Nickel-Autoklaven wurden bei 140 °C 775 g 80 %ige wäßrige Natronlauge ( = 15,5 Mol) vorgelegt. In den Autoklaven wurden 408 g Diphenyl-4,4'-disulfonsäure-Sulfiergemisch (0,5 Mol Diphenyl-4,4'-disulfonsäure) folgender Zusammensetzung :

| | | |
|---|---|---|
| | 38,5 | Gew.-% Diphenyl-4,4'-disulfonsäure |
| ca. | 1,0 | Gew.-% Diphenyl-3,4'-disulfonsäure |
| ca. | 0,2 | Gew.-% Diphenyl-3,3'-disulfonsäure |
| | 0,05 | Gew.-% Diphenyl-4-monosulfonsäure |
| | 55,8 | Gew.-% $H_2SO_4$ |
| | 4,5 | Gew.-% $H_2O$ |

das auf 120 °C erhitzt wurde, eingepumpt. Die Temperatur stieg auf ca. 325 °C, der Druck auf 40 bar an. Die Reaktionsmischung wurde 14 h bei 325 °C nachgerührt, auf 200 °C abgekühlt, mit 400 ml Wasser verdünnt, auf 20 °C abgekühlt und mit Wasser bis zur Lösung auf ein Volumen von ca. 3 Ltr. verdünnt. Die Lösung wurde durch Zugabe von ca. 410 g (4,2 Mol) 100-Gew.-%iger $H_2SO_4$ auf einen pH-Wert von 7 eingestellt, die Suspension auf 20 °C abgekühlt, das ausgefallene 4,4'-Dihydroxydiphenyl filtriert, mit ca. 1 000 ml Wasser gewaschen und im Vakuum bei 60 °C getrocknet. Man erhielt 73,5 g 96,5 %iges 4,4'-Dihydroxydiphenyl = 92 % der theoretischen Ausbeute, bezogen auf Diphenyl-4,4'-disulfonsäure. Das Produkt enthielt 1,3 Gew.-% 4-Hydroxy-diphenyl.

**Patentansprüche**

1. Verfahren zur Herstellung von Hydroxydiphenylen der Formel

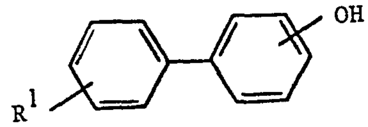

in der
$R^1$ Wasserstoff oder Hydroxyl bedeutet,
durch Umsetzung von Diphenylsulfonsäuren der Formel

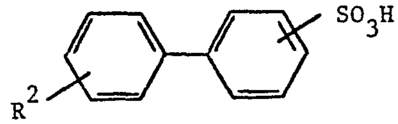

in der
$R^2$ Wasserstoff oder die Sulfonsäuregruppe bedeutet,
oder ihrer Alkalisalze mit Alkalihydroxid bei erhöhter Temperatur und erhöhtem Druck in Gegenwart von Wasser, dadurch gekennzeichnet, daß man bei einer Temperatur zwischen 280 und 330 °C und einem erhöhten Druck von bis zu 120 bar 3 bis 25 Mol wäßriges Alkalihydroxid mit einer Konzentration von mindestens 50 Gew.-% pro Äquivalent Sulfonatgruppe einsetzt.

2. Verfahren nach Anspruch 1, dadurch gekennzeichnet, daß man bei 300 bis 325 °C arbeitet.

3. Verfahren nach Anspruch 1 und 2, dadurch gekennzeichnet, daß man bei einem Druck von 5 bis 80 bar arbeitet.

4. Verfahren nach Anspruch 1 und 2, dadurch gekennzeichnet, daß man bei einem Druck von 10 bis 40 bar arbeitet.

5. Verfahren nach Anspruch 1 bis 4, dadurch gekennzeichnet, daß man 50 bis 96 gew.-%ige wäßrige Alkalihydroxidlösung einsetzt.

6. Verfahren nach Anspruch 1 bis 5, dadurch gekennzeichnet, daß man 5 bis 12 Mol Alkalihydroxid pro Äquivalent Sulfonatgruppe einsetzt.

7. Verfahren nach Anspruch 1 bis 6, dadurch gekennzeichnet, daß als Alkalihydroxid Natriumhydroxid eingesetzt wird.

8. Verfahren nach Anspruch 1 bis 7, dadurch gekennzeichnet, daß man

a) ein technisches Sulfiergemisch einsetzt, das neben 1 bis 60 Gew.-% Schwefelsäure und gegebenenfalls neben 3 bis 30 Gew.-% Wasser, alles bezogen auf das Gesamtgewicht des Gemisches, mindestens 80 Gew.-% Diphenylsulfonsäuren der in Anspruch 1 genannten Formel, bezogen auf das Gewicht der insgesamt vorhandenen organischen Bestandteile, enthält,

b) dieses Gemisch mit soviel Alkalihydroxid versetzt, daß nach Neutralisation aller vorhandenen Säuregruppen 3 bis 25 Mol Alkalihydroxid pro Äquivalent Sulfonatgruppe vorliegen,

c) diese alkalische Mischung bei Temperaturen im Bereich zwischen 280 und 330 °C und bei einem Druck von bis zu 120 bar umsetzt, und

d) die alkalische Reaktionsmischung in an sich bekannter Weise, gegebenenfalls nach Verdünnen mit Wasser, mit Mineralsäuren bis zu einem pH-Wert von kleiner als 8 versetzt und das Hydroxydiphenyl der in Anspruch 1 genannten Formel bei einer Temperatur von − 5 bis + 100 °C isoliert.

9. Verfahren nach Anspruch 8, dadurch gekennzeichnet, daß man ein technisches Sulfiergemisch in vorgelegtes waßriges Alkalihydroxid unter Druck einpumpt und unter Ausnutzung der freiwerdenden Wärmemengen die Umsetzung zum Hydroxydiphenyl durchführt.

10. Verfahren nach Anspruch 8, dadurch gekennzeichnet, daß man ein technisches Sulfiergemisch und wäßriges Alkalihydroxid in einem Mischrohr mischt und gegebenenfalls mindestens teilweise darin zum Hydroxydiphenyl umsetzt.

**Claims**

1. Process for the preparation of hydroxydiphenyls of the formula

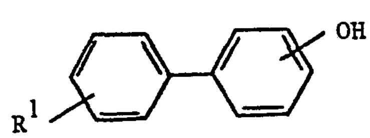

in which
$R^1$ denotes hydrogen or hydroxyl,
by reacting diphenylsulphonic acids of the formula

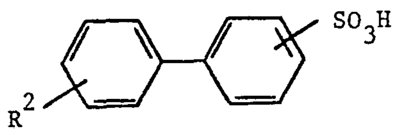

in which

$R^2$ denotes hydrogen or the sulphonic acid group,

or their alkali metal salts, with an alkali metal hydroxide at elevated temperature and elevated pressure, in the presence of water, characterised in that 3 to 25 mols of aqueous alkali metal hydroxide having a concentration of at least 50 % by weight are employed per equivalent of sulphonate group, at a temperature between 280 and 330 °C and an elevated pressure of up to 120 bar.

2. Process according to Claim 1, characterised in that the reaction is carried out at 300 to 325 °C.

3. Process according to Claims 1 and 2, characterised in that the reaction is carried out at a pressure of 5 to 80 bar.

4. Process according to Claims 1 and 2, characterised in that the reaction is carried out at a pressure of 10 to 40 bar.

5. Process according to Claims 1 to 4, characterised in that 50 to 96 % strength by weight aqueous alkali metal hydroxide solution is employed.

6. Process according to Claims 1 to 5, characterised in that 5 to 12 mols of alkali metal hydroxide are employed per equivalent of sulphonate group.

7. Process according to Claims 1 to 6, characterised in that sodium hydroxide is employed as the alkali metal hydroxide.

8. Process according to Claims 1 to 7, characterised in that

a) an industrial sulphonating mixture which contains, in addition to 1 to 60 % by weight of sulphuric acid and if appropriate in addition to 3 to 30 % by weight of water, all relative to the total weight of the mixture, at least 80 % by weight of diphenylsulphonic acids of the formula mentioned in Claim 1, relative to the weight of the total organic constituents present, is employed,

b) an amount of alkali metal hydroxide is added to this mixture such that, after neutralisation of all acid groups present, 3 to 25 mols of alkali metal hydroxide per equivalent of sulphonate group are present,

c) this alkaline mixture is reacted at temperatures in the range between 280 and 330 °C and at a pressure of up to 120 bar, and

d) mineral acids are added to the alkaline reaction mixture in a manner which is in itself known, if appropriate after dilution with water, the addition being carried out until a pH value of less than 8 is obtained, and the hydroxydiphenyl of the formula mentioned in Claim 1 is isolated at a temperature of − 5 to + 100 °C.

9. Process according to Claim 8, characterised in that an industrial sulphonating mixture is pumped, under pressure, into initially introduced aqueous alkali metal hydroxide, and the reaction to give the hydroxydiphenyl is carried out utilising the amounts of heat liberated.

10. Process according to Claim 8, characterised in that an industrial sulphonating mixture and aqueous alkali metal hydroxide are mixed in a mixing tube, and if necessary are at least partially reacted therein to give hydroxydiphenyl.

**Revendications**

1. Procédé pour la fabrication d'hydroxydiphényles de formule

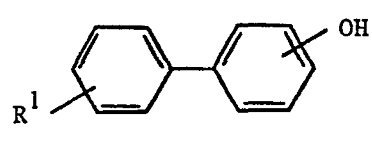

dans laquelle

$R^1$ représente l'hydrogène ou un hydroxyle,

par réaction d'acides diphénylesulfoniques de formule

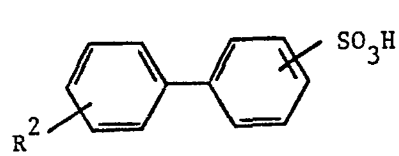

dans laquelle

$R^2$ représente l'hydrogène ou le groupe acide sulfonique,

ou de leurs sels alcalins avec un hydroxyde alcalin à température élevée et sous pression élevée en présence d'eau, caractérisé en ce que l'on utilise 3 à 25 mol d'hydroxyde alcalin aqueux à une concentration d'au moins 50 % en poids par équivalent de groupe sulfonate à une température entre 280 et 330 °C et sous une pression élevée jusqu'à 120 bars.

2. Procédé selon la revendication 1, caractérisé en ce que l'on opère à 300-325 °C.

3. Procédé selon les revendications 1 et 2, caractérisé en ce que l'on opère sous une pression de 5 à 80 bars.

4. Procédé selon les revendications 1 et 2, caractérisé en ce que l'on opère sous une pression de 10 à 40 bars.

5. Procédé selon les revendications 1 à 4, caractérisé en ce que l'on utilise une solution aqueuse d'hydroxyde alcalin à 50-96 % en poids.

6. Procédé selon les revendications 1 à 5, caractérisé en ce que l'on utilise 5 à 12 mol d'hydroxyde alcalin par équivalent de groupe sulfonate.

7. Procédé selon les revendications 1 à 6, caractérisé en ce que l'on utilise comme hydroxyde alcalin d'hydroxyde de sodium.

8. Procédé selon les revendications 1 à 7, caractérisé en ce que

a) on utilise un mélange technique de sulfonation qui, outre 1 à 60 % en poids d'acide sulfurique et éventuellement 3 à 30 % en poids d'eau, calculés sur le poids total du mélange, contient au moins 80 % en poids d'acides diphénylesulfoniques de la formule mentionnée à la revendication 1, par rapport au poids total des composants organiques présents,

b) on ajoute à ce mélange suffisamment d'hydroxyde alcalin pour qu'il y ait après neutralisation de tous les groupes acides présents 3 à 25 mol d'hydroxyde alcalin par équivalent de groupe sulfonate,

c) on fait réagir ce mélange alcalin à des températures dans l'intervalle de 280 à 330 °C et sous une pression allant jusqu'à 120 bars, et

d) on ajoute de manière connue au mélange de réaction alcalin, éventuellement après dilution par l'eau, des acides minéraux jusqu'à un pH de moins de 8 et on isole l'hydroxydiphényle de la formule mentionnée à la revendication 1 à une température de − 5 à + 100 °C.

9. Procédé selon la revendication 8, caractérisé en ce que l'on introduit sous pression par pompage un mélange de sulfonation technique dans l'hydroxyde alcalin aqueux préalablement chargé et l'on effectue la transformation en hydroxydiphényle avec exploitation des quantités de chaleur libérées.

10. Procédé selon la revendication 8, caractérisé en ce que l'on mélange dans un tube de mélange un mélange de sulfonation technique et l'hydroxyde alcalin aqueux et éventuellement on les y fait réagir au moins partiellement en hydroxydiphényle.